# EUROPÄISCHE PATENTSCHRIFT

(11) **EP 1 543 863 B1**
(45) Veröffentlichungstag und Bekanntmachung des Hinweises auf die Patenterteilung: **09.09.2009**
(21) Anmeldenummer: 04027736.0
(22) Anmeldetag: 23.11.2004
(51) Int. Cl.: A61N 1/05, A61N 1/04

(54) **Elektrodenstruktur, Verfahren zu ihrer Herstellung und ihre Verwendung**
Electrode structure, methods of manufacture and use of it
Structure d'életrode, procédés de sa fabrication et son utilisation

(30) Priorität: 19.12.2003 DE 10360624
(43) Veröffentlichungstag der Anmeldung: 22.06.2005
(73) Patentinhaber: W.C. Heraeus GmbH, 63450 Hanau (DE)
(72) Erfinder: Specht, Heiko, 63739 Aschaffenburg (DE); Krüger, Frank Dr., 63486 Bruchköbel (DE)
(74) Vertreter: Kühn, Hans-Christian

(56) Entgegenhaltungen:
- WO-A-02/087685
- US-A- 5 324 322
- US-A- 5 683 443
- US-A- 5 720 099
- US-B1- 6 374 143

## Beschreibung

Die Erfindung betrifft eine Elektrodenstruktur aus einem Edelmetall oder einer Edelmetall-Legierung mit einer Dicke von ≤ 100 µm für Implantate sowie drei Verfahren zu deren Herstellung. Die Erfindung betrifft weiterhin die Verwendung einer derartigen Elektrodenstruktur.

Elektrodenanordnungen aus Edelmetall sind beispielsweise aus der WO 02/089907 A1 bekannt. Dabei werden Elektrodenstrukturen aus Platinfolie gebildet und auf einem Träger aus Kunststoffmaterial fixiert. Derartige Elektrodenanordnungen finden beispielsweise Verwendung für Cochlearimplantate.

Die US 6,266,568 B1 offenbart eine aufweitbare Cochlea-Elektrodenanordnung und ein Verfahren zu deren Herstellung. Dabei sind auf einem flexiblen Träger eine Vielzahl von zueinander beabstandet angeordneten Elektrodenkontakten angeordnet.

Die DE-OS 35 07 623 A1 offenbart eine langzeitimplantierbare Flächenelektrode mit einer physiologisch inerten Matrix aus flexiblem Kunststoff, welche mit einem elektrischen Leiter, beispielsweise aus Platin, beschichtet wird. Die Beschichtung erfolgt dabei galvanisch oder mittels Bedampfen.

Eine Noch-dichte Anordnung von Elektroden zur neuronalen Stimulation ist aus WO 02/87685 bekannt. Eine Einzelelektrode weist einen An- oder Pt-Kern auf, das von Pt-ummantelt ist. Dieses Dokument beschreibt auch ein Verfahren zur Herstellung dieser Elektrodenanordnung.

Es ist nun Aufgabe der Erfindung, eine weitere Elektrodenstruktur für Implantate zur Verfügung zu stellen, welche einfach und kostengünstig hergestellt werden kann. Weiterhin sollen geeignete Verfahren zu deren Herstellung sowie eine Verwendung angegeben werden.

Die Aufgabe wird durch die Elektrodenstruktur nach Anspruch 1 gelöst.

Eine derartige Elektrodenstruktur ist kostengünstig, da sie einen gut leitfähigen, duktilen Elektrodenkern aus kostengünstigerem Material aufweist, welcher lediglich mit einer dünnen, teureren Beschichtung ummantelt ist, welche korrosionsbeständig und biokompatibel ist. Gegebenenfalls kann zwischen dem Elektrodenkern und der ersten Beschichtung durch einen Temperaturprozess eine Legierungsbildung herbeigeführt werden.

Es hat sich bewährt, wenn der Elektrodenkern eine Dicke im Bereich von etwa 5 µm bis 99,8 µm aufweist. Für die erste Beschichtung hat sich eine Dicke im Bereich von etwa 100 nm bis 5 µm bewährt. Besonders bevorzugt ist es, die Elektrodenstruktur mit einer Dicke im Bereich von etwa 7 µm bis 30 µm auszubilden.

Vorzugsweise wird auf der dem Elektrodenkern abgewandten Seite der ersten Beschichtung eine zur ersten Beschichtung unterschiedliche zweite Beschichtung aus Ruthenium, Rutheniumoxid, Iridium, Iridiumoxid, Platin oder Titannitrid gebildet. Dabei hat es sich bewährt, wenn die zweite Beschichtung lediglich Teilbereiche der ersten Beschichtung bedeckt.

Die Aufgabe wird für ein erstes Verfahren dadurch gelöst, dass
a) eine elektrisch isolierende Oberfläche eines Substrats mit einer Metallschicht beschichtet wird, dass die Metallschicht anschließend mit Photoresist beschichtet wird und dass der Photoresist strukturiert wird, dass
b) auf den nun freiliegenden Bereichen der Metallschicht galvanisch ein erster Teil der ersten Beschichtung erzeugt wird, dass
c) auf dem ersten Teil der ersten Beschichtung galvanisch der Elektrodenkern gebildet wird, dass nun
d) der strukturierte Photoresist von der Metallschicht entfernt wird, dass
e) die Metallschicht in den Bereichen, die nicht von dem ersten Teil der ersten Beschichtung bedeckt sind, von der elektrisch isolierenden Oberfläche des Substrats entfernt wird, dass
f) der bislang noch von der ersten Beschichtung freie Teil des Elektrodenkems galvanisch mit einem zweiten Teil der ersten Beschichtung beschichtet wird, dass
g) die Elektrodenstruktur inklusive der Metallschicht von der elektrisch isolierenden Oberfläche des Substrats abgelöst wird, und dass nun
h) die Metallschicht von der Elektrodenstruktur entfernt wird.

Die Aufgabe wird für ein zweites Verfahren dadurch gelöst, dass
a) eine elektrisch isolierende Oberfläche eines Substrats mit einer Maske versehen wird und auf den nicht mit der Maske bedeckten Bereichen der elektrisch isolierenden Oberfläche einer Metallschicht mittels Kathodenzerstäubung oder Aufdampfen gebildet wird, dass
b) ein erster Teil der ersten Beschichtung mittels Kathodenzerstäubung oder Aufdampfen auf der Metallschicht erzeugt wird, dass
c) auf dem ersten Teil der ersten Beschichtung nun der Elektrodenkern mittels Kathodenzerstäubung oder Aufdampfen gebildet wird, dass dann
f) die Maske entfernt wird, und
g) zumindest der bislang noch von der ersten Beschichtung freie Teil des Elektrodenkerns galvanisch mit einem zweiten Teil der ersten Beschichtung beschichtet wird, dass nun
h) die Elektrodenstruktur von der elektrisch isolierenden Oberfläche des Substrats abgelöst wird, indem die Metallschicht entfernt wird.

Die Aufgabe wird für ein drittes Verfahren dadurch gelöst, dass
a) eine elektrisch isolierende Oberfläche eines Substrats mit einer strukturierten Metallschicht beschichtet wird, welche einen ersten Teil der ersten Beschichtung bildet, dass
b) der erste Teil der ersten Beschichtung mit einer Maske versehen wird und nur auf dem ersten Teil der ersten Beschichtung mittels Kathodenzerstäubung oder Aufdampfen der Elektrodenkem gebildet wird, dass nun
c) der bislang noch von der ersten Beschichtung freie Teil des Elektrodenkerns galvanisch mit einem zweiten Teil der ersten Beschichtung beschichtet wird, und dass nun
d) die Elektrodenstruktur von der elektrisch isolierenden Oberfläche des Substrats abgelöst wird.

Derartige Verfahren eignen sich hervorragend zur kostengünstigen und schnellen Herstellung der erfindungsgemäßen Elektrodenstruktur.

Der Elektrodenkem kann dabei mit mehreren unterschiedlichen Beschichtungen umhüllt werden. Zur Bildung einer zweiten Beschichtung auf der ersten Beschichtung muss dann beispielsweise bei dem dritten Verfahren die elektrisch isolierende Oberfläche eines Substrats mit einer strukturierten Metallschicht beschichtet werden, welche einen ersten Teil der zweiten Beschichtung bildet und darauf dann der erste Teil der ersten Beschichtung gebildet werden. Nach Bildung des Elektrodenkems und des zweiten Teils der ersten Beschichtung wird schließlich ein zweiter Teil der zweiten Beschichtung gebildet. Entsprechende Masken sind jeweils zu verwenden.

Dabei hat es sich für die drei erfindungsgemäßen Verfahren insbesondere bewährt, die elektrisch isolierende Oberfläche des Substrats aus Glas oder Kunststoff zu bilden. Eine geeignete Metallschicht kann vorzugsweise aus Kupfer oder Gold gebildet sein.

Eine Verwendung der erfindungsgemäßen Elektrodenstruktur für eine Stimulations-, Cochleaoder Retinaelektrode ist ideal.

Die Figuren 1a bis 3g sollen die erfindungsgemäße Elektrodenstruktur sowie die Herstellung einer solchen beispielhaft erläutern. So zeigt:
- Fig. 1: mehrere Prinzipdarstellungen von Elektrodenstrukturen mit elektrischen Zuleitungen in der Draufsicht,
- Fig. 1a: die Prinzipdarstellungen der Elektrodenstrukturen aus Fig. 1 im Schnittbild A-A',
- Fig. 2a bis 2d: ein drittes Verfahren zu Herstellung der erfindungsgemäßen Elektrodenstruktur, und
- Fig. 3a bis 3h: ein erstes Verfahren zu Herstellung der erfindungsgemäßen Elektrodenstruktur.

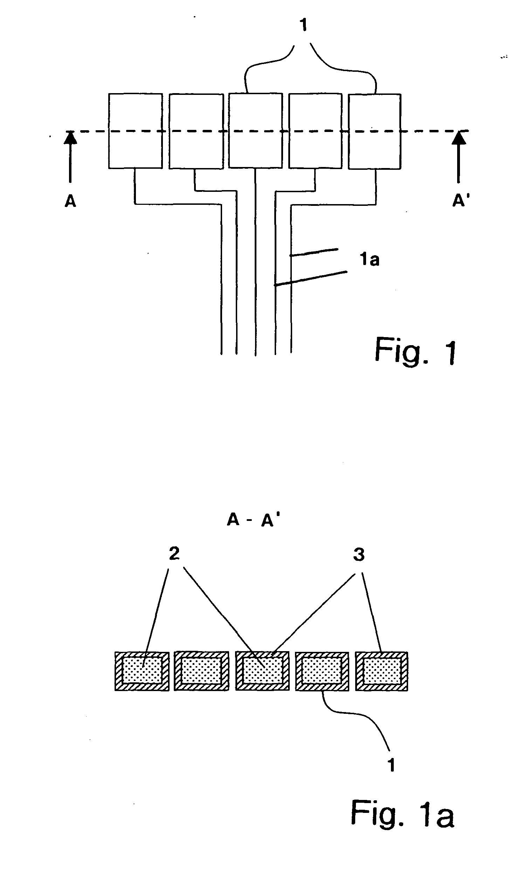

Figur 1 zeigt mehrere Elektrodenstrukturen 1 in einer hier willkürlich gewählten Anordnung zueinander. Jede Elektrodenstruktur 1 ist mit einer elektrischen Leitung 1a verbunden. Selbstverständlich kann aber auch jegliche andere Anordnung gewählt werden.

Figur 1a zeigt den Schnitt A - A' aus Figur 1. Dabei ist erkennbar, dass eine Elektrodenstruktur 1 aus einem Elektrodenkern 2 und einer ersten Beschichtung 3, welche den Elektrodenkern 2 vollständig umschließt, aufgebaut ist.

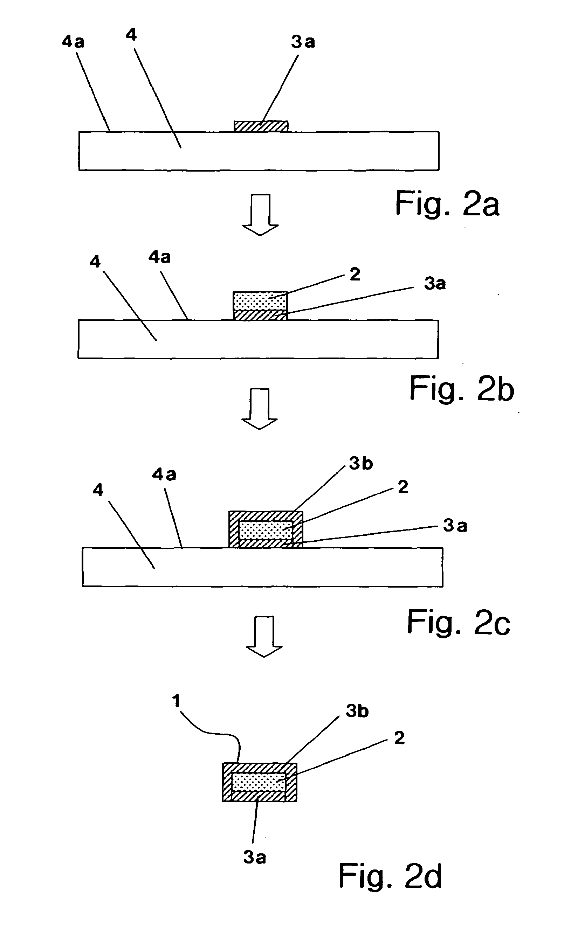

Figuren 2a bis 2d zeigen den Ablauf eines dritten erfindungsgemäßen Verfahrens zur Herstellung einer erfindungsgemäßen Elektrodenstruktur 1. In Figur 2a wird dabei eine elektrisch isolierende Oberfläche 4a eines Substrats 4 mit einem ersten Teil einer ersten Beschichtung 3a aus Iridium bedampft. Figur 2b verdeutlicht, dass auf dem ersten Teil der ersten Beschichtung 3a ein Elektrodenkern 2 aus Gold aufgedampft wurde. Figur 2c zeigt, dass auf dem Elektrodenkern 2 ein zweiter Teil der ersten Beschichtung 3b aus Iridium galvanisch abgeschieden wurde. Die vom Substrat 4 gelöste, fertiggestellte Elektrodenstruktur 1 zeigt schließlich Figur 2d. Die für das Aufdampfen des ersten Teils der ersten Beschichtung 3a und den Elektrodenkern 2 erforderlichen Masken, die die daneben liegenden Bereiche des Substrats 4 abdecken, sind nicht dargestellt.

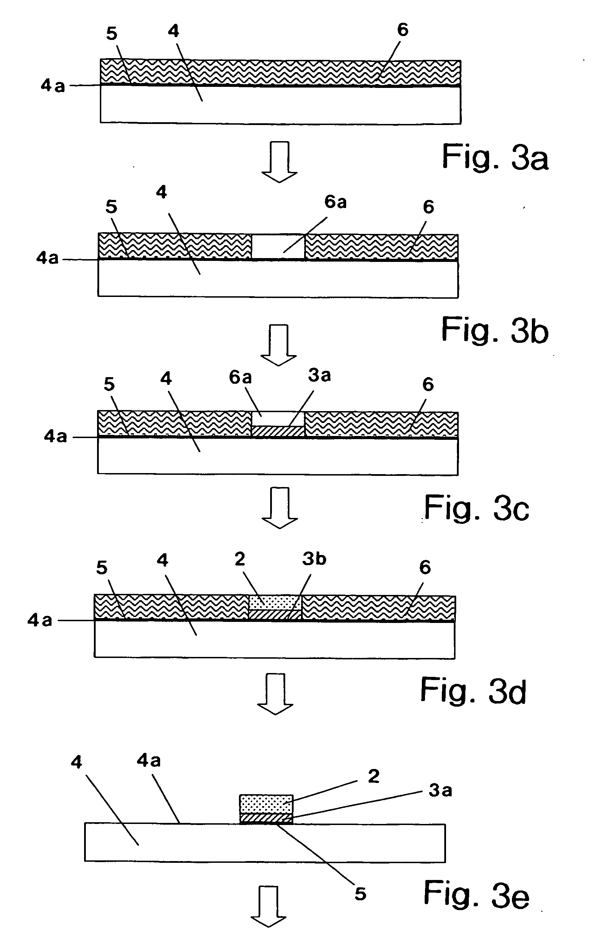

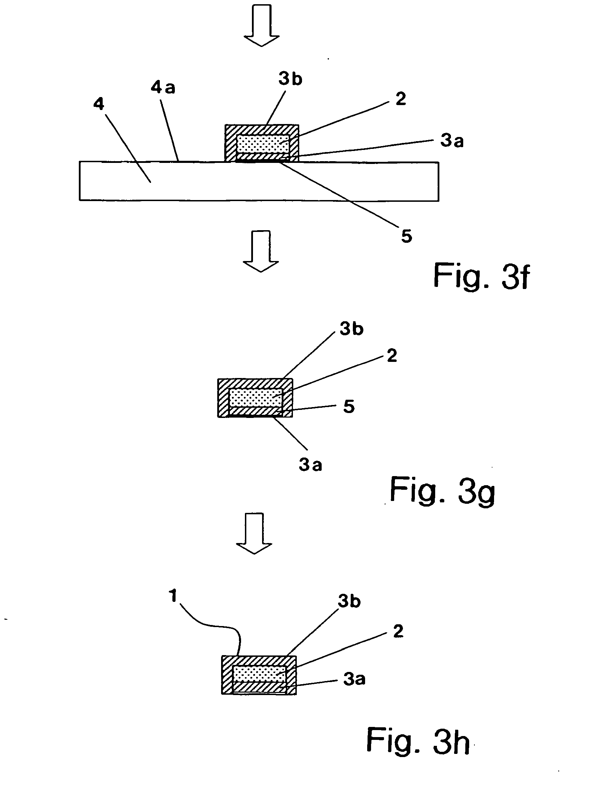

Figuren 3a bis 3h zeigen den Ablauf eines ersten erfindungsgemäßen Verfahrens zur Herstellung einer erfindungsgemäßen Elektrodenstruktur 1. In Figur 3a wird dabei eine elektrisch isolierende Oberfläche 4a aus Glas eines Substrats 4 mit einer Metallschicht 5 aus Kupfer und einem Photoresist 6 beschichtet. Der Photoresist 6 wird derart strukturiert, dass die Metallschicht 5 teilweise über Öffnungen 6a im Photoresist 6 freigelegt wird (siehe Figur 3b ). In der Öffnung 6a wird nun ein erster Teil einer ersten Beschichtung 3a aus Platin galvanisch abgeschieden ( siehe Figur 3c ). Figur 3d verdeutlicht, dass nun auf dem ersten Teil der ersten Beschichtung 3a ein Elektrodenkem 2 aus Kupfer galvanisch abgeschieden wurde. Figur 3e zeigt, dass nun der Photoresist 6 sowie die danach freiliegenden Teile der Metallschicht 5 entfernt werden. Nun wird ein zweiter Teil der ersten Beschichtung 3b aus Platin galvanisch erzeugt ( siehe Figur 3f ). Nach Entfernung des Substrats 4 verbleibt an der Elektrodenstruktur noch ein Teil der Metallschicht 5 ( siehe Figur 3g ), die noch entfernt wird. Die fertiggestellte Elektrodenstruktur 1 mit dem Elektrodenkern 2 und einer ersten Beschichtung 3a, 3b zeigt schließlich Figur 3h.

## Patentansprüche

1. Elektrodenstruktur (1) aus einem Edelmetall oder einer Edelmetall-Legierung mit einer Dicke von ≤ 100µm für Implantate bestehend aus einem Elektrodenkern (2) und ein beschichtung (3;3a;3b), wobei der Elektrodenkern (2) aus Gold, Silber, Kupfer oder einer Legierung aus mindestens zwei dieser Elemente besteht und vollständig von der ersten Beschichtung (3; 3a, 3b) die aus Platin, Iridium oder Ruthenium gebildet ist ummantelt ist.

2. Elektrodenstruktur (1) nach Anspruch 1, **dadurch gekennzeichnet, dass** der Elektrodenkern (2) eine Dicke im Bereich von 5µm bis 99,8µm aufweist.

3. Elektrodenstruktur (1) nach einem der Ansprüche 1 bis 2, **dadurch gekennzeichnet, dass** die erste Beschichtung (3; 3a, 3b) eine Dicke im Bereich von 100nm bis 5µm aufweist.

4. Elektrodenstruktur (1) nach einem der Ansprüche 1 bis 3, **dadurch gekennzeichnet, dass** die Elektrodenstruktur (1) eine Dicke im Bereich von 7µm bis 30µm aufweiset.

5. Elektrodenstruktur (1) nach einem der Ansprüche 1 bis 4, **dadurch gekennzeichnet, dass** auf der dem Elektrodenkern (2) abgewandten Seite der ersten Beschichtung (3; 3a, 3b) eine zur ersten Beschichtung (3; 3a, 3b) unterschiedliche zweite Beschichtung aus Ruthenium, Rutheniumoxid, Iridium, Iridiumoxid, Platin oder Titannitrid gebildet ist.

6. Elektrodenstruktur (1) nach Anspruch 5, **dadurch gekennzeichnet, dass** die zweite Beschichtung lediglich Teilbereiche der ersten Beschichtung (3; 3a, 3b) bedeckt.

7. Verfahren zur Herstellung einer Elektrodenstruktur (1) nach einem der Ansprüche 1 bis 6, wobei
a) eine elektrisch isolierende Oberfläche (4a) eines Substrats (4) mit einer Metallschicht (5) beschichtet wird, dass die Metallschicht (5) anschließend mit Photoresist (6) beschichtet wird und dass der Photoresist (6) strukturiert wird, dass
b) auf den nun freiliegenden Bereichen der Metallschicht (5) galvanisch ein erster Teil der ersten Beschichtung (3a) erzeugt wird, dass
c) auf dem ersten Teil der ersten Beschichtung (3a) galvanisch der Elektrodenkern (2) gebildet wird, dass nun
d) der strukturierte Photoresist (6) von der Metallschicht (5) entfernt wird, dass
e) die Metallschicht (5) in den Bereichen, die nicht von dem ersten Teil der ersten Beschichtung (3a) bedeckt sind, von der elektrisch isolierenden Oberfläche (4a) des Substrats (4) entfernt wird, dass
f) der bislang noch von der ersten Beschichtung (3a, 3b) freie Teil des Elektrodenkerns (2) galvanisch mit einem zweiten Teil der ersten Beschichtung (3b) beschichtet wird, dass
g) die Elektrodenstruktur (1) inklusive der Metallschicht (6) von der elektrisch isolierenden Oberfläche (4a) des Substrats (4) abgelöst wird, **dadurch gekennzeichnet dass**, nun
h) die Metallschicht (5) von der Elektrodenstruktur (1) entfernt wird.

8. Verfahren zur Herstellung einer Elektrodenstruktur (1) nach einem der Ansprüche 1 bis 6, wobei
a) eine elektrisch isolierende Oberfläche (4a) eines Substrats (4) mit einer Maske versehen wird und auf den nicht mit der Maske bedeckten Bereichen der elektrisch isolierenden Oberfläche (4a) eine Metallschicht (5) mittels Kathodenzerstäubung oder Aufdampfen gebildet wird, dass
b) ein erster Teil der ersten Beschichtung (3a) mittels Kathodenzerstäubung oder Aufdampfen auf der Metallschicht (5) erzeugt wird, dass
c) auf dem ersten Teil der ersten Beschichtung (3a) nun der Elektrodenkern (2) mittels Kathodenzerstäubung oder Aufdampfen gebildet wird, dass dann
f) die Maske entfernt wird, und
g) zumindest der bislang noch von der ersten Beschichtung (3a, 3b) freie Teil des Elektrodenkerns (2) galvanisch mit einem zweiten Teil der ersten Beschichtung (3b) beschichtet wird, **dadurch gekennzeichnet dass** nun
h) die Elektrodenstruktur (1) von der elektrisch isolierenden Oberfläche (4a) des Substrats (4) abgelöst wird, indem die Metallschicht (5) entfernt wird.

9. Verfahren zur Herstellung einer Elektrodenstruktur (1) nach einem der Ansprüche 1 bis 6, , wobei
a) eine elektrisch isolierende Oberfläche (4a) eines Substrats (4) mit einer strukturierten Metallschicht beschichtet wird, welche einen ersten Teil der ersten Beschichtung (3a) bildet, dass
b) der erste Teil der ersten Beschichtung (3a) mit einer Maske versehen wird und nur auf dem ersten Teil der ersten Beschichtung (3a) mittels Kathodenzerstäubung oder Aufdampfen der Elektrodenkern (2) gebildet wird, dass nun
c) der bislang noch von der ersten Beschichtung (3a) freie Teil des Elektrodenkerns (2) galvanisch mit einem zweiten Teil der ersten Beschichtung (3b) beschichtet wird,
**dadurch gekennzeichnet, dass** nun
d) die Elektrodenstruktur (1) von der elektrisch isolierenden Oberfläche (4a) des Substrats (4) abgelöst wird.

10. Verfahren nach einem der Ansprüche 7, 8 oder 9, **dadurch gekennzeichnet, dass** die elektrisch isolierende Oberfläche (4a) des Substrats (4) aus Glas oder Kunststoff gebildet ist.

11. Verfahren nach Anspruch 7 oder 8, **dadurch gekennzeichnet, dass** die Metallschicht (5) aus Kupfer oder Gold gebildet ist.

12. Verwendung einer Elektrodenstruktur (1) nach einem der Ansprüche 1 bis 6 für Stimulations-, Cochlea- oder Retinaelektroden.

## Claims

1. Electrode structure (1) consisting of a noble metal or a noble metal alloy with a thickness of ≤ 100 µm for implants consisting of an electrode core (2) and a coating (3, 3a, 3b), the electrode core (2) consisting of gold, silver, copper or an alloy of at least two of these elements and being encased completely by the first coating (3, 3a, 3b) which is formed of platinum, iridium or ruthenium.

2. Electrode structure (1) according to claim 1 **characterised in that** the electrode core (2) has a thickness within the range of 5 µm to 99.8 µm.

3. Electrode structure (1) according to one of claims 1 to 2 **characterised in that** the first coating (3, 3a, 3b) has a thickness within the range of 100 nm to 5 µm.

4. Electrode structure (1) according to one of claims 1 to 3 **characterised in that** the electrode structure has a thickness within the range of 7 µm to 30 µm.

5. Electrode structure (1) according to one of claims 1 to 4 **characterised in that**, on the side of the first coating (3, 3a, 3b) facing away from the electrode core (2), a second coating of ruthenium, ruthenium oxide, iridium, iridium oxide, platinum or titanium nitride, which is different from the first coating (3, 3a, 3b), is formed.

6. Electrode structure (1) according to claim 5 **characterised in that** the second coating covers merely partial areas of the first coating (3, 3a, 3b).

7. Process for the production of an electrode structure (1) according to one of claims 1 to 6 in which
a) an electrically insulating surface (4a) of a substrate (4) is coated with a metal layer (5), the metal layer (5) is subsequently coated with photoresist (6) and the photoresist (6) is structured
b) a first part of the first coating (3a) is produced galvanically on the now exposed areas of the metal layer (5)
c) the electrode core (2) is formed galvanically on the first part of the first coating (3a),
d) the structured photoresist (6) is now removed from the metal layer (5),
e) the metal layer (5) is removed from the electrically insulating surface (4a) of the substrate (4) in the areas not covered by the first part of the first coating (3a),
f) the part of the electrode core (2) so far free from the first coating (3a, 3b) is coated galvanically with a second part of the first coating (3b),
g) the electrode structure (1) including the metal layer (5) is detached from the electrically insulating surface (4a) of the substrate (4) **characterised in that**
h) the metal layer (5) is now removed from the electrode structure (1).

8. Process for the production of an electrode structure (1) according to one of claims 1 to 6 in which
a) an electrically insulating surface (4a) of a substrate (4) is provided with a mask and, on the areas of the electrically insulating surface (4a) not covered with the mask, a metal layer (5) is formed by cathode sputtering or vapour deposition,
b) a first part of the first coating (3a) is produced on the metal layer (5) by cathode sputtering or vapour deposition,
c) on the first part of the first coating (3a), only the electrode core (2) is formed by cathode sputtering or vapour deposition,
f) the mask is then removed and
g) at least the part of the electrode core (2) so far free from the first coating (3a, 3b) is coated galvanically with a second part of the first coating (3b)
**characterised in that**
h) the electrode structure (1) is now detached from the electrically insulating surface (4a) of the substrate (4) by the metal layer (5) being removed.

9. Process for the production of an electrode structure (1) according to one of claims 1 to 6 in which
a) an electrically insulating surface (4a) of a substrate (4) is coated with a structured metal layer which forms a first part of the first coating (3a)
b) the first part of the first coating (3a) is provided with a mask and the electrode core (2) is formed only on the first part of the first coating (3a) by cathode sputtering or vapour deposition,
c) the part of the electrode core (2) so far free from the first coating (3a) is coated galvanically with a second part of the first coating (3b),
**characterised in that**
d) the electrode structure (1) is now detached from the electrically insulating surface (4a) of the substrate (4).

10. Process according to one of claims 7, 8 or 9 **characterised in that** the electrically insulating surface (4a) of the substrate (4) is formed of glass or plastic.

11. Process according to claim 7 or 8 **characterised in that** the metal layer (5) is formed of copper or gold.

12. Use of an electrode structure (1) according to one of claims 1 to 6 for stimulation electrodes, cochlear electrodes or retinal electrodes.

## Revendications

1. Structure d'électrode (1) en métal noble ou alliage de métal noble d'une épaisseur ≤ 100 µm pour implants consistant en un coeur d'électrode (2) et en un revêtement (3 ; 3a, 3b), où le coeur d'électrode (2) consiste en or, en argent, en cuivre ou en un alliage d'au moins deux de ces éléments et est entouré totalement par le premier revêtement (3; 3a, 3b) qui est formé de platine, d'iridium ou de ruthénium.

2. Structure d'électrode (1) selon la revendication 1 **caractérisée en ce que** le coeur d'électrode (2) présente une épaisseur dans le domaine de 5 µm à 99,8 µm.

3. Structure d'électrode (1) selon l'une des revendications 1 à 2 **caractérisée en ce que** le premier revêtement (3 ; 3a, 3b) présente une épaisseur dans le domaine de 100 nm à 5 µm.

4. Structure d'électrode (1) selon l'une des revendications 1 à 3 **caractérisée en ce que** la structure d'électrode (1) présente une épaisseur dans le domaine de 7 µm à 30 µm.

5. Structure d'électrode (1) selon l'une des revendications 1 à 4 **caractérisée en ce qu'**un second revêtement différent du premier revêtement (3; 3a, 3b), en ruthénium, oxyde de ruthénium, iridium, oxyde d'iridium, platine ou nitrure de titane est formé sur le côté du premier revêtement (3; 3a, 3b) opposé au coeur d'électrode (2).

6. Structure d'électrode (1) selon la revendication 5 **caractérisée en ce que** le second revêtement couvre seulement des domaines partiels du premier revêtement (3; 3a, 3b).

7. Procédé de production d'une structure d'électrode (1) selon l'une des revendications 1 à 6 où
a) une surface électriquement isolante (4a) d'un substrat (4) est recouverte d'une couche métallique (5), la couche métallique (5) est ensuite recouverte d'un photorésist (6) et le photorésist (6) est structuré,
b) une première partie du premier revêtement (3a) est formée sur les domaines maintenant libres de la couche métallique (5) par galvanisation,
c) le coeur d'électrode (2) est formé par galvanisation sur la première partie du premier revêtement (3a),
d) le photorésisl structuré (6) est alors retiré de la couche métallique (5),
e) la couche métallique (5) est retirée de la surface électriquement isolante (4a) du substrat (4) dans les domaines qui ne sont pas recouverts par la première partie du premier revêtement (3a),
f) la partie du coeur d'électrode (2) qui est encore dépourvue du premier revêtement (3a, 3b) est recouverte par galvanisation d'une seconde partie du premier revêtement (3b),
g) la structure d'électrode (1), y compris la couche métallique (5), est séparée de la surface électriquement isolante (4a) du substrat (4), **caractérisé en ce que**
h) la couche métallique (5) est alors retirée de la structure d'électrode (1).

8. Procédé de production d'une structure d'électrode (1) selon l'une des revendications 1 à 6 où
a) une surface électriquement isolante (4a) d'un substrat (4) est munie d'un masque et une couche métallique (5) est formée par pulvérisation cathodique ou métallisation sous vide sur les domaines de la surface électriquement isolante (4a) qui ne sont pas recouverts par le masque,
b) une première partie du premier revêtement (3a) est produite par pulvérisation cathodique ou métallisation sous vide sur la couche métallique (5),
c) le coeur d'électrode (2) est alors formé par pulvérisation cathodique ou métallisation sous vide sur la première partie du premier revêtement (3a),
f) le masque est ensuite retiré, et
g) au moins la partie du coeur d'électrode (2) qui est encore dépourvue du premier revêtement (3a, 3b) est recouverte par galvanisation d'une seconde partie du premier revêtement (3b), **caractérisé en ce que**
h) la structure d'électrode (1) est alors séparée de la surface électriquement isolante (4a) du substrat (4), **en ce que** la couche métallique (5) est retirée.

9. Procédé de production d'une structure d'électrode (1) selon l'une des revendications 1 à 6 où
a) une surface électriquement isolante (4a) d'un substrat (4) est recouverte d'une couche métallique structurée qui forme une première partie du premier revêtement (3a),
b) la première partie du premier revêtement (3a) est munie d'un masque et le coeur d'électrode (2) est formé seulement sur la première partie du premier revêtement (3a) par pulvérisation cathodique ou métallisation sous vide,
c) la partie du coeur d'électrode (2) qui est encore dépourvue du premier revêtement (3a) est recouverte d'une seconde partie du premier revêtement (3b) par galvanisation,
**caractérisé en ce que**
d) la structure d'électrode (1) est alors séparée de la surface électriquement isolante (4a) du substrat (4).

10. Procédé selon l'une des revendications 7, 8 ou 9 **caractérisé en ce que** la surface électriquement isolante (4a) du substrat (4) est formée de verre ou de manière synthétique.

11. Procédé selon la revendication 7 ou 8 **caractérisé en ce que** la couche métallique (5) est formée de cuivre ou d'or.

12. Utilisation d'une structure d'électrode (1) selon l'une des revendications 1 à 6 pour des électrodes de stimulation, de cochlée ou de rétine.
